(19) Europäisches Patentamt
European Patent Office
Office européen des brevets

(11) Veröffentlichungsnummer: **0 162 016 B1**

(12) # EUROPÄISCHE PATENTSCHRIFT

(45) Veröffentlichungstag der Patentschrift: **03.06.92**

(51) Int. Cl.$^5$: **C10M 135/24**, C08K 5/37

(21) Anmeldenummer: **85810221.3**

(22) Anmeldetag: **09.05.85**

(54) **Zusätze für Materialien.**

(30) Priorität: **15.05.84 CH 2388/84**

(43) Veröffentlichungstag der Anmeldung:
**21.11.85 Patentblatt 85/47**

(45) Bekanntmachung des Hinweises auf die Patenterteilung:
**03.06.92 Patentblatt 92/23**

(84) Benannte Vertragsstaaten:
**BE DE FR GB IT**

(56) Entgegenhaltungen:
**FR-M- 3 854**

(73) Patentinhaber: **CIBA-GEIGY AG**
**Klybeckstrasse 141**
**CH-4002 Basel(CH)**

(72) Erfinder: **Wirth, Hermann O. Dr.**
**Lessingstrasse 24**
**W-6140 Bensheim 3(DE)**
Erfinder: **Friedrich, Hans-Helmut**
**Am Rauhenstein 8**
**W-6147 Lautertal 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft Zusammensetzungen, enthaltend Aldosemercaptale, einen Kunststoff, einen Schmierstoff oder eine Hydraulikflüssigkeit, die auf Oel oder Wasser oder Gemischen davon basieren können, die Verwendung der Aldosemercaptale sowie neue Aldosemercaptale.

Einige Aldosemercaptale und deren Herstellung sind aus der Literatur bekannt, z.B. aus Chem.Ber. 27, 673 (1894) oder aus Chem. Ber. 84, 780 (1951); diese dienten der Identifizierung und Isolierung von Aldosen. Die Anwendung bestimmter Vertreter ist für spezielle Gebiete in US 3 635 717 und US 3 635 736 beschrieben; sie werden dort einerseits eingesetzt, um eine verbesserte licht-empfindliche photographische Silberhalogenid-Emulsion zu liefern, und andererseits als Aromastoffe in Nahrungsmitteln.

Aus der FR-PS 3854 M (Brévet spécial de Médicament) sind Aldosemercaptale als pharmazeutisch wirksame Substanzen in Tierkörpern bestimmter Gattungen bekannt.

Um die Gebrauchseigenschaften von Schmierstoffen zu verbessern und deren natürlichen oder technisch bedingten Alterungserscheinungen entgegenzuwirken, werden den Schmierstoffen Additive beigegeben. Die beispielsweise bei erhöhter Temperatur verstärkt durch Luftsauerstoff eintretenden Oxidationsreaktionen in einem Schmierstoff können durch Zugabe von Antioxidantien unterbunden werden. Detergentien werden beispielsweise eingesetzt, weil sie u.a. in Motorenölen, ölunlösliche Oxidations- und Verbrennungsrückstände in Suspensionen halten und dadurch Ablagerungen an Metalloberflächen und Schlammausscheidungen im Motor und Verschleiss durch Neutralisation saurer Verbrennungsprodukte verhindern. Weiterhin soll der Schmierstoff bei hohen Drücken zwischen den Kontakten, wie z.B. Zahnrädern, Nocken-Stössel-Systemen, Fresserscheinungen auf den Metalloberflächen verhindern; dieses bewirken sog. Hochdruck- und Antiverschleiss-Additive.

Wenn andererseits z.B. Sauerstoff und Feuchtigkeit gleichzeitig auf eine Metalloberfläche einwirken, kann Korrosion auftreten, weshalb Korrosionsinhibitoren mit dem Ziel zugegeben werden, den Zutritt dieser Stoffe zur Metalloberfläche zu verhindern. Um beispielsweise Oelverluste durch Abtropfen ober Abspritzen zu verhindern, setzt man sog. Haftverbesserer zu. Es ist bekannt, dass bestimmte Stoffe als Additive für Schmierstoffe eine Anzahl von solchen Eigenschaften in sich vereinigen; sie werden als sog. Vielzweck-Additive bezeichnet. Solche Stoffe sind - wie sich leicht vorstellen lässt - aus ökonomischen und praktischen Gründen natürlich sehr gefragt.

Wenn PVC thermisch belastet wird, wie es z.B. bei der thermoplastischen Verformung geschieht, kann eine Schädigung durch Chlorwasserstoffabspaltung (Dehydrochlorierung),Autoxidation und mechanochemische Fragmentierung auftreten. Um PVC bei der Verarbeitung möglichst nicht zu schädigen und um seine Gebrauchseigenschaften zu erhalten, setzt man spezifische Stabilisatoren zu, welche einerseits die Dehydrochlorierung und Autoxidation verzögern (präventive Stabilisatorfunktionen), aber auch bereits eingetretene Schäden wenigstens teilweise beheben (curative Stabilisatorfunktionen).

Es ist bekannt, dass natürliche und synthetische Polymere der Oxidation durch Sauerstoff unterliegen; diese kann während der gesamten Lebenszeit eines Polymeren in unerwünschter Weise wirksam werden. Die damit verbundenen Alterungserscheinungen sind einerseits ästhetischer Art und andererseits erfolgt mehr oder weniger parallel dazu auch ein Verlust an mechanischen Eigenschaften. Bei den in die Polymeren einzuarbeitenden Antioxidantien handelt es sich um chemische Verbindungen, welche die Oxidation und die daraus resultierenden Alterungserscheinungen eines Polymeren verzögern können.

Da die meisten Kunststoffe aufgrund ihrer chemischen Konstitution ausgesprochene Isolatoren sind, werden sie z.B. auch als Werkstoffe in der Hochfrequenztechnik eingesetzt. Nachteilig ist dabei, dass einmal aufgebrachte Ladungen wegen der geringen Oberflächenleitfähigkeit nicht rasch genug abgeführt werden können, was zu einer hohen Aufladung führt. Durch Antistatika kann die Oberflächenleitfähigkeit verbessert werden, so dass die Ladungen über die Oberfläche abgeleitet werden können.

Die vorliegende Erfindung betrifft nun Zusammensetzungen, enthaltend A) einen Schmierstoff, einen Kunststoff, oder eine Hydraulikflüssigkeit und B) eine Verbindung der Formel I

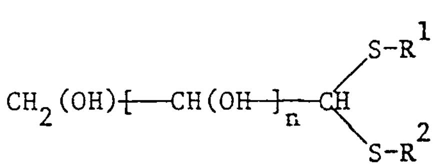

(I),

worin n eine natürliche Zahl von 2 bis 6 sein kann und worin $R^1$ und $R^2$ gleich oder verschieden sind und jeweils unsubstituiertes, substituiertes oder durch -O- oder -S- unterbrochenes $C_1$-$C_{18}$ Alkyl, $-(CH_2)_r$-CO-N-

($C_1$-$C_{17}$ Alkyl)$_2$ mit r gleich 1 oder 2, Phenyl, Benzyl oder -$(CH_2)_r$-CO-O-$R^3$ bedeuten, wobei r gleich 1 oder 2 sein kann und $R^3$ Alkalimetall oder $C_1$-$C_{14}$ Alkyl darstellt, ferner worin $R^1$ und $R^2$ -$CH_2$-CH(OH)-$R^4$ sind, wobei $R^4$ Wasserstoff, unsubstituiertes oder durch -OH substituiertes $C_1$-$C_{16}$ Alkyl oder -$CH_2$-Y-($C_1$-$C_{15}$ Alkyl) mit Y gleich -O- oder -S- ist, oder worin $R^1$ und $R^2$ zusammen -$(CH_2)_m$- bilden, wobei m eine natürliche Zahl von 2 bis 4 sein kann.

Sind $R^1$ und/oder $R^2$ $C_1$-$C_{18}$ Alkyl, so handelt es sich um geradkettige oder verzweigte Substituenten, wie beispielsweise Methyl, Ethyl, n-Propyl, Isopropyl, n-Butyl, sec-Butyl, tert-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl, insbesondere aber um geradkettiges oder verzweigtes Octyl, Nonyl, Decyl, Undecyl oder Dodecyl, Pentadecyl oder Hexadecyl und im speziellen um 2-Ethyl-hexyl, 2-Butyl-octyl oder 2-Hexyl-decyl sowie um isoprenoides Decyl oder Pentadecyl.

Bei $C_1$-$C_{17}$ Alkyl und $C_1$-$C_{15}$ Alkyl handelt es sich um geradkettige und verzweigte Substituenten, wie beispielsweise Methyl, Ethyl, n-Propyl, Isopropyl, n-Butyl, sec-Butyl, tert-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl. Bei $C_1$-$C_{15}$ Alkyl sind Hexadecyl und Heptadecyl nicht eingeschlossen.

Dreht es sich bei $R^3$ in -$(CH_2)_r$-CO-O-$R^3$, welches als Substituent für $R^1$ und/oder $R^2$ fungieren kann, um Alkalimetall, so handelt es sich um Lithium, Natrium oder Kalium, insbesondere aber um Natrium oder Kalium; dreht es sich bei $R^3$ um $C_1$-$C_{14}$ Alkyl, so handelt es sich um geradkettige oder verzweigte Substituenten, wie beispielsweise Methyl, Ethyl, n-Propyl, Isopropyl, n-Butyl, sec-Butyl, tert-Butyl, Pentyl, Hexyl, Heptyl, Octyl, Nonyl, Decyl, Undecyl, Dodecyl, Tridecyl oder Tetradecyl.

Dreht es bei $R^4$ in -$CH_2$-CH(OH)-$R^4$, welches als Substituent für $R^1$ und/oder $R^2$ fungieren kann, um $C_1$-$C_{16}$ Alkyl, so handelt es sich um geradkettige oder verzweigte Substituenten, wie beispielsweise Methyl, Ethyl, n-Propyl, Isopropyl, n-Butyl, sec-Butyl, tert-Butyl, Pentyl,Hexyl, Heptyl,Octyl, tert.-Nonyl,Decyl,Undecyl,tert.-Dodecyl,Tridecyl, Tetradecyl, Pentadecyl oder Hexadecyl. Ist $C_1$-$C_{16}$ Alkyl als $R^4$ durch -OH substituiert, so sind alle Monohydroxy-Stellungsisomeren möglich, es können jedoch auch mehrfache Substitutionen durch -OH auftreten, bevorzugt zweifache.

Bilden $R^1$ und $R^2$ zusammen als -$(CH_2)_m$- mit den S-Atomen, an die sie gebunden sind, einen heterocyclischen Ring, so handelt es sich beispielsweise um einen Ring mit 5 bis 7 Ringatomen, bevorzugt um einen Ring mit 5 oder 6 Ringatomen.

Bevorzugt ist organisches Material, enthaltend eine Verbindung der Formel I, worin n eine natürliche Zahl von 3 bis 6 sein kann, und worin $R^1$ und $R^2$ gleich sind und unsubstituiertes oder durch -O- oder -S- unterbrochenes $C_1$-$C_{16}$ Alkyl, Phenyl, Benzyl oder -$(CH_2)_r$-CO-O-$R^3$bedeuten, wobei r gleich 1 oder 2 sein kann und $R^3$ Alkalimetall ist, oder worin $R^1$ und $R^2$ -$CH_2$-CH(OH)-$R^4$ sind, wobei $R^4$ Wasserstoff oder $C_1$-$C_{14}$ Alkyl oder -$CH_2$-O-($C_1$-$C_{13}$ Alkyl) ist, oder worin $R^1$ und $R^2$ zusammen -$(CH_2)_m$- bilden, wobei m eine natürliche Zahl von 2 bis 4 sein kann.

Besonders bevorzugt ist organisches Material, enthaltend eine Verbindung der Formel I, worin n eine natürliche Zahl von 3 bis 6 sein kann, und worin $R^1$ und $R^2$ gleich sind und $C_8$-$C_{16}$ Alkyl oder -$CH_2$-O-($C_7$-$C_{15}$ Alkyl), Phenyl, Benzyl oder -$CH_2$-COOK, -$CH_2$-COONa oder -$CH_2$-$CH_2$-COOK bedeuten oder worin $R^1$ und $R^2$ -$CH_2$-CH(OH)-$R^4$ sind, wobei $R^4$ Wasserstoff oder $C_6$-$C_{14}$ Alkyl oder -$CH_2$-O-($C_5$-$c_{13}$ Alkyl) ist, oder worin $R^1$ und $R^2$ zusammen -$(CH_2)_m$- bilden, wobei m eine natürliche Zahl von 2 bis 4 sein kann.

Einen weiteren Gegenstand der Erfindung stellen Verbindungen der Formel I dar, worin n eine natürliche Zahl von 2 bis 6 sein kann, und worin $R^1$ und $R^2$ gleich oder verschieden sind und jeweils verzweigtes $C_5$-$C_{18}$ Alkyl, -$(CH_2)_r$-CO-O-$R^3$, wobei r gleich 1 oder 2 sein kann und $R^3$ Alkalimetall oder $C_1$-$C_{14}$ Alkyl darstellt, oder -$CH_2$-CH(OH)-$R^4$ bedeuten, wobei $R^4$ Wasserstoff oder unsubstituiertes oder durch -OH substituiertes $C_1$-$C_{16}$ Alkyl oder -$CH_2$-Y-($C_1$-$C_{15}$ Alkyl) mit Y gleich -O- oder -S- darstellt, insbesondere aber -$(CH_2)_r$-CO-O$R^3$ oder -$CH_2$-CH(OH)-$R^4$ bedeuten.

Bevorzugt sind Verbindungen der Formel I, worin n eine natürliche Zahl von 3 bis 6 sein kann, und worin $R^1$ und $R^2$ gleich sind und -$CH_2$-CH-[$(CH_2)_q$-$CH_3$][$(CH_2)_{q+2}$-$CH_3$], wobei q gleich 1 bis 5 sein kann, -$(CH_2)_r$-CO-O-$R^3$, wobei r gleich 1 oder 2 sein kann und $R^3$ Alkalimetall ist, oder -$CH_2$-CH(OH)-$R^4$ bedeuten, wobei $R^4$ Wasserstoff oder $C_1$-$C_{14}$ Alkyl oder -$CH_2$-O-($C_1$-$C_{13}$ Alkyl) bedeutet.

Besonders bevorzugt sind Verbindungen der Formel I, worin n eine natürliche Zahl von 4 bis 6 sein kann, und worin $R^1$ und $R^2$ gleich sind und -2-Ethyl-hexyl, -2-Butyl-octyl, -2-Hexyl-decyl, -$CH_2$-COOK, -$CH_2$-COONa oder -$CH_2$-$CH_2$-COOK bedeuten oder worin $R^1$ und $R^2$ -$CH_2$-CH(OH)-$R^4$ sind, wobei $R^4$ Wasserstoff oder $C_6$-$C_{14}$ Alkyl oder -$CH_2$-O-($C_5$-$C_{13}$ Alkyl) darstellt.

Die bereits gegebene beispielhafte Bedeutung von $R^3$ und $R^4$ soll auch hier für die neuen Aldosemercaptale der Formel I gelten.

Als Beispiele für neue Verbindungen der Formel I sind hier die folgenden aufgeführt:

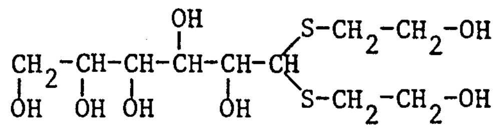

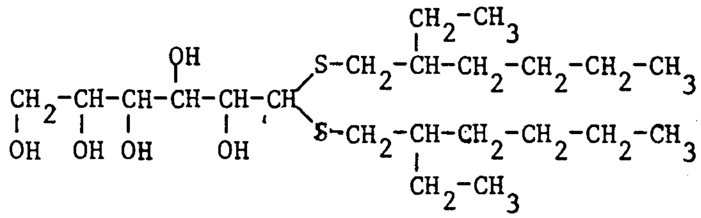

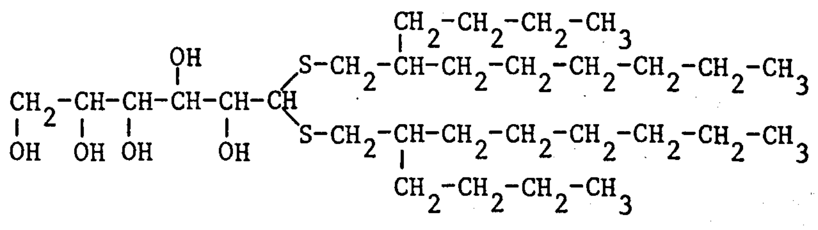

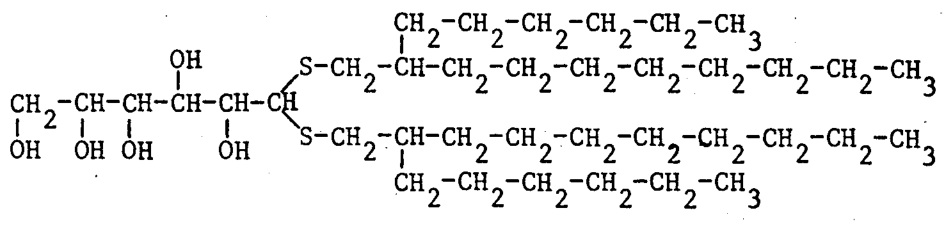

Die Herstellung der bekannten und neuen Aldosemercaptale der Formel I geschieht nach an sich bekannten Methoden. Synthesemethoden sind unter anderem in Chem. Ber. 27, 673 (1984), J.A.C.S. 53, 4 379 (1931), Chem. Ber. 49, 2056 (1916) oder Chem.Ber. 84, 780 (1951) beschrieben.

Besonders vorteilhaft für die Synthese von Aldosemercaptale ist die Verwendung eines Ionenaustauscherharzes in der H-Form.

Die Aldosemercaptale der Formel I sind als Additive für eine Reihe von Substraten geeignet. An dieser Stelle soll spezifisch jedoch nur auf die Verwendung in Schmierstoffen, Hydraulikflüssigkeiten und Kunststoffen eingegangen werden.

Dem Schmierstoff werden 0,001-5,0 Gew.-%, bevorzugt jedoch 0,005-3,0 Gew.-% und besonders bevorzugt 0,1-1,0 Gew.-% des Aldosemercaptals der Formel I, bezogen auf das Gewicht der Schmierstoff-Formulierung zugesetzt.

Sind mineralische und synthetische Schmieröle, hydraulische Flüssigkeiten und Schmierfette auf solche Weise ausgerüstet, so zeigen sie ausgezeichnete Eigenschaften, was sich in stark reduzierten Abnutzungserscheinungen der zu schmierenden Teile bemerkbar macht; dieses ist darauf zurückzuführen, dass die Aldosemercaptale der Formel I als Vielzweck-Additive die antioxidierende, antikorrosive und dispergierende Wirkung in den Schmierstoffen steigern; vor allem verbessern sie das Hochdruck-Verhalten und die Antiverschleiss-Wirksamkeit; als Haftverbesserer beugen sie Oelverlusten vor.

Die in Frage kommenden Schmierstoffe sind dem Fachmann geläufig und z.B. im "Schmiermittel-Taschenbuch (Hüthig Verlag, Heidelberg, 1974)" beschrieben. Besonders geeignet sind neben Mineralölen z.B. Poly-α-Olefine, Schmierstoffe auf Esterbasis, Phosphate, Glykole, Polyglykole und Polyalkylenglykole.

Die Schmierstoff-Formulierungen können zusätzlich noch andere Additive enthalten, die zugegeben werden, um weitere Gebrauchs-Eigenschaften zu verbessern, wie z.B. Antioxidantien, Metallpassivatoren, Rostinhibitoren, Viskositätsindex-Verbesserer, Stockpunkterniedriger, Dispergiermittel/Tenside und Verschleisschutz-Additive. Als Beispiele sind zu nennen:

Beispiele für phenolische Antioxidantien

1. Alkylierte Monophenole

2,6-Di-tert.-butyl-4-methylphenol
2,6-Di-tert.-butylphenol
2-tert.-butyl-4,6-Dimethylphenyl

2,6-Di-tert.-butyl-4-ethylphenol
2,6-Di-tert.-butyl-4-ethylphenol
2,6-Di-tert.-butyl-4-n-butylphenol
2,6-Di-tert.-butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.-butyl-4-methoxymethylphenyl
o-tert.-Butylphenol

2. Alkylierte Hydrochinone

2,6-Di-tert.-butyl-4-methoxyphenol
2,5-Di-tert.-butyl-hydrochinon
2,5-Di-tert.-amyl-hydrochinon
2,6-Diphenyl-4-octadecyloxyphenol

3. Hydroxylierte Thiodiphenylether

2,2'-Thio-bis-(6-tert.-butyl-4-methylphenol)
2,2'-Thio-bis-(4-octylphenol)
4,4'-Thio-bis-(6-tert.-butyl-3-methylphenol)
4,4'-Thio-bis-(6-tert.-butyl-2-methylphenol)

4. Alkyliden-Bisphenole 2,2'-Methylen-bis-(6-tert.-butyl-4-methylphenol)

2,2'-Methylen-bis-(6-tert.-butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.-butylphenol)
2,2'-Ethyliden-bis-(4,6-di-tert.-butylphenol)
2,2'-Ethyliden-bis-(6-tert.-butyl-4-isobutylphenol)
2,2'-Methylen-bis-[6-(α-methylbenzyl)-4-nonylphenol]
2,2'-Methylen-bis-[6-[α,α-dimethylbenzyl)-4-nonylphenol]
4,4'-Methylen-bis-(2,6-di-tert.-butylphenol)
4,4'-Methylen-bis-(6-tert.-butyl-2-methylphenol)
1,1-Bis-(5-tert.-butyl-4-hydroxy-2-methylphenyl)-butan
2,6-Di-(3-tert.-butyl-5-methyl-2-hydroxybenzyl)-4-methylphenol
1,1,3-Tris-(5-tert.-butyl-4-hydroxy-2-methylphenyl)-3-n-dodecyl-mercaptobutan
Ethylenglycol-bis-[3,3-bis-(3'-tert.-butyl-4'-hydroxyphenyl)-butyrat]
Di-(3-tert.-butyl-4-hydroxy-5-methylphenyl)-dicyclopentadien
Di-[2-(3'-tert.-butyl-2'-hydroxy-5'-methyl-benzyl)-6-tert.-butyl-4-methyl-phenyl]-terephthalat.

5. Benzylverbindungen

1,3,5-Tri-(3,5-di-tert.-butyl-4-hydroxybenzyl)-2,4,6-trimethylbenzol
Di-(3,5-di-tert.-butyl-4-hydroxybenzyl)-sulfid
3,5-Di-tert.butyl-4-hydroxybenzyl-mercaptoessigsäure-isooctylester
Bis-(4-tert.-butyl-3-hydroxy-2,6-dimethylbenzyl)dithiol-terephthalat
1,3,5-Tris-(3,5-di-tert.-butyl-4-hydroxybenzyl)-isocyanurat
1,3,5-Tris-(4-tert.-butyl-3-hydroxy-2,6-dimethylbenzyl)-isocyanurat
3,5-Di-tert.-butyl-4-hydroxybenzyl-phosphonsäure-dioctadecylester
3,5-Di-tert.-butyl-4-hydroxybenzyl-phosphonsäure-monoethylester
Calcium-salz.

6. Acylaminophenole 4-Hydroxy-laurinsäureanilid

4-Hydroxy-stearinsäureanilid
2,4-Bis-octylmercapto-6-(3,5-di-tert.-butyl-4-hydroxyanilino)-s-triazin
N-(3,5-di-tert.-butyl-4-hydroxyphenyl)-carbaminsäureoctylester.

7. Ester der β-(3,5-Di-tert.-butyl-4-hydroxyphenyl)-propionsäure mit ein- oder mehrwertigen Alkoholen, wie z.B. mit

| | |
|---|---|
| Methanol<br>Octadecanol<br>1,6-Hexandiol<br>Neopentylglycol<br>Thiodiethylenglycol | Diethylenglycol<br>Triethylenglycol<br>Pentaerythrit<br>Tris-hydroxyethyl-isocyanurat<br>Di-hydroxyethyl-oxalsäurediamid |

8. Ester der $\beta$-(5-tert.-butyl-4-hydroxy-3-methylphenyl)-propionsäure mit ein- oder mehrwertigen Alkoholen, wie z.B. mit

| | |
|---|---|
| Methanol<br>Octadecanol<br>1,6-Hexandiol<br>Neopentylglycol<br>Thiodiethylenglycol | Diethylenglycol<br>Triethylenglycol<br>Pentaerythrit<br>Tris-hydroxyethyl-isocyanurat<br>Di-hydroxyethyl-oxalsäurediamid |

9. Amide der $\beta$-(3,5-Di-tert.-butyl-4-hydroxyphenyl)-propionsäure, wie z.B.
N,N'-Di-(3,5-di-tert.-butyl-4-hydroxyphenylpropionyl)-hexamethylendiamin
N,N'-Di-(3,5-di-tert.-butyl-4-hydroxyphenylpropionyl)-trimethylendiamin
N,N'-Di-(3,5-di-tert.-butyl-4-hydroxyphenylpropionyl)-hydrazin.

Beispiele für aminische Antioxidantien:

N,N'-Di-isopropyl-p-phenylendiamin
N,N'-Di-sec.-butyl-p-phenylendiamin
N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-p-phenylendiamin
N,N'-Dicyclohexyl-p-phenylendiamin
N,N'-Diphenyl-p-phenylendiamin
N,N'-Di-(naphthyl-2-)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec.-butyl-p-phenylendiamin
Diphenylamin
4-Isopropoxy-diphenylamin
N-Phenyl-1-naphthylamin
N-Phenyl-2-naphthylamin
tert.-octyliertes N-Phenyl-1-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert.-butyl-4-dimethylamino-methyl-phenol
2,4'-Diamino-diphenylmethan
4,4'-Diamino-diphenylmethan
N,N,N,N'-Tetramethyl-4,4'-diamino-diphenylmethan
1,2-Di-(phenylamino)-ethan
1,2-Di-[(2-methyl-phenyl)-amino]-ethan
1,3-Di-(phenylamino)-propan
(o-Tolyl)-biguanid

Di-[4-(1',3'-dimethyl-butyl)-phenyl)amin
tert.-octyliertes N-Phenyl-1-naphthylamino
Gemisch aus mono- und dialkylierten tert.-Butyl-/tert.-Octyl-diphenylaminen.

Beispiele für Metallpassivatoren sind:

für Kupfer, z.B.:
Benztriazol, Tetrahydrobenztriazol, 2-Mercaptobenzthiazol 2,5-Dimercaptothiadiazol, Salicyliden-propylendiamin, Salze von Salicylaminoguanidin.

Beispiele für Rost-Inhibitoren sind:

a) Organische Säuren, ihre Ester, Metallsalze und Anhydride, z.B.: N-Oleoyl-sarcosin, Sorbitan-monooleat, Blei-naphthenat, Dodecenylbernsteinsäure-anhydrid, Alkenylbernsteinsäure-Halbester, 4-Nonylphenoxy-essigsäure.
b) Stickstoffhaltige Verbindungen, z.B.:
  I. Primäre, sekundäre oder tertiäre aliphatische oder cycloaliphatische Amine und Amin-Salze von organischen und anorganischen Säuren, z.B. öllösliche Alkylammoniumcarboxylate.
  II. Heterocyclische Verbindungen z.B.: Substituierte Imidazoline und Oxazoline.
c) Phosphorhaltige Verbindungen, z.B.: Aminsalze von Phosphorsäurepartialestern.
d) Schwefelhaltige Verbindungen, z.B.: Barium-dinonylnaphthalin-sulfonate, Calciumpetroleum-sulfonate.

Beispiele für Viskositätsindex-Verbesserer sind z.B.

Polymethacrylate, Vinylpyrrolidon/Methacrylat-Copolymere, Polybutene, Olefin-Copolymere, Styrol/Acrylat-Copolymere.

Beispiele für Stockpunktniedriger sind z.B.:

Polymethacrylat, alkylierte Naphthalinderivate.

Beispiele für Dispergiermittel/Tenside sind z.B.:

Polybutenylbernsteinsäure-imide, Polybutenylphosphonsäurederivate, basische Magnesium-, Calcium-, und Bariumsulfonate und -phenolate.

Beispiele für Verschleissschutz-Additive sind z.B.:

Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Aryldisulfide.

Weiterhin können die Aldosemercaptale der Formel I auch als Zusätze für Kunststoffe und Elastomere verwendet werden, worin sie als Thermostabilisatoren und Antioxidantien, z.B. für PVC, und als Haftvermittler, speziell für Metall, Glas, Holz, Keramit und Beton, wirksam sind. Ausserdem weisen sie eine gute Antistatika-Wirksamkeit auf.

Die Aldosemercaptale der Formel I werden den Kunststoffen in einer Konzentration von 0,005 bis 5 Gew.-%, bezogen auf das Gewicht des zu stabilisierenden Kunststoffes, zugesetzt. Vorzugsweise werden 0,05-2 Gew.-% und insbesondere 0,1 bis 2 Gew.-% der Aldosemercaptale der Formel I, bezogen auf das zu stabilisierende Material, in dieses eingearbeitet.

Die Einarbeitung kann beispielsweise durch Einmischen der Verbindungen der Formel I und gegebenenfalls weiterer Additive nach den in der Technik üblichen Methoden, vor oder während der Formgebung,oder auch durch Aufbringen der gelösten oder dispergierten Verbindungen auf das Polymere, gegebenenfalls unter nachträglichem Verdunsten des Lösungsmittels, erfolgen. Die Einarbeitung kann separat oder als Gemisch durchgeführt werden.

Die Aldosemercaptale der Formel I können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyethylen werden die erfindungsgemässen Zusammensetzungen vor der Vernetzung beigefügt.

Die so stabilisierten Materialien können in verschiedenster Form angewendet werden, z.B. als Folien, insbesondere Agrofolien, Fasern, Bändchen, Formmassen, Profile, geschäumte Artikel oder als Bindemittel für Lacke, Klebstoffe oder Kitte.

Als Beispiele für Kunststoffe, die mit den Aldosemercaptalen der Formel I ausgerüstet werden können, seien genannt:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

5. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 4) genannten Copolymeren, wie sie z.B. als sogenannte ABS, MBS, ASA oder AES-Polymere bekannt sind.

6. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

7. Copolymere von Monomeren wie $\alpha,\beta$-ungesättigte Säuren und deren Derivate mit andern ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

8. Homo- und Copolymere von cyclischen Ethern, wie Polyalkylenglykole, Polyethylenoxyd, Polypropylenoxyd oder deren Copolymere mit Bisglycidylethern.

9. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

10. Natürliche Polymere, wie Cellulose, Naturkautschuk, Gelatine, sowie deren polymerhomolog chemisch abgewandelte Derivate, wie Celluloseacetate, -propionate und -butyrate, bzw. die Cellulose-ether, wie Methylcellulose.

11. Mischungen (Polyblends) der vorgenannten Polymeren wie z.B. PP/EPDM, Polyamid 6/EPDM oder ABS, PVC/EVA, PVC/ABS, PVC/MBS, PC/ABS, PBTP/ABS.

12. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Spinnpräparationen Anwendung finden, sowie deren wässrigen Emulsionen.

13. Wässrige Emulsionen natürlicher oder synthetischer Kautschuke, wie z.B. Naturkautschuk-Latex oder Latices von carboxylierten Styrol-Butadien-Copolymeren.

In der Praxis können die Aldosemercaptale der Formel I zusammen mit anderen Stabilisatoren eingesetzt werden. Als weitere Additive, mit denen die Aldosemercaptale zusammen eingesetzt werden können, sind beispielsweise zu nennen:

1. Antioxidantien

1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole

2,2'-Methylen-bis-(6-tert.butyl-4-methylphenol)
2,2'-Methylen-bis(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methlyen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5 Benzylverbindungen

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

1.6. Acylaminophenole

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit

| | |
|---|---|
| Methanol<br>Octadecanol<br>1,6-Hexandiol<br>Neopentylglycol<br>Thiodiethylenglycol | Diethylenglycol<br>Triethylenglycol<br>Pentaerythrit<br>Tris-hydroxyethyl-isocyanurat<br>Di-hydroxyethyl-oxalsäurediamid |

1.8. Ester der β-(5-tert.butyl-4-hydroxy-3-methylphenyl)-propionsäure mit ein- oder mehrwertigen Alkoholen, wie z.B. mit

| | |
|---|---|
| Methanol<br>Octadecanol<br>1,6-Hexandiol<br>Neopentylglycol<br>Thiodiethylenglycol | Diethylenglycol<br>Triethylenglycol<br>Pentaerythrit<br>Tris-hydroxyethyl-isocyanurat<br>Di-hydroxyethyl-oxalsäurediamid |

1.9. Amide der β-(3,5-Di-tert.butyl-4-hydroxyphenyl)-propionsäure, wie z.B.
N,N'-Di-(3,5-di-tert.butyl-4-hydroxyphenylpropionyl)-hexamethylendiamin
N,N'-Di-(3,5-di-tert.-butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z.B. das 5'-Methyl-, 3',5'-Di-tert.butyl-, 5'-Tert.butyl-, 5'-(1,1,3,3-Tetramethylbutyl)-,5-Chlor-3',5'-di-tert.butyl-, 5-Chlor-3'-tert.butyl-5'-methyl-,3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyl-dithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6 Sterisch gehinderte Amine, wie z.B. Bis-(2,2,6,6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-Tetramethyl-4-hydroxypiperidin und Bernsteinsäure, Kondensationsprodukt aus N,N'-(2,2,6,6-Tetramethylpiperidyl)-hexamethylendiaminund 4-tert.octylamino-2,6-dichlor-1,3,5-s-triazin,
Tris-(2,2,6,6-tetramethylpiperidyl)-nitrilotriacetat,
Tetrakis-(2,2,6,6-tetramethyl-4-piperidyl)-1,2,3,4-butantetracarbonsäure,
1,1'-(1,2-ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Aldosemercaptale der Formel I eignen sich als Zusätze zu mineralischen und synthetischen Schmierölen, hydraulischen Flüssigkeiten, die auf Oel oder Wasser oder Gemischen davon basieren können, und Schmierfetten. Sie können als Vielzweck-Additive eingesetzt werden und wirken speziell gegen Korrosion und Oxidation, verbessern die Hochdruck-, Verschleissschutz und Dispergier-Eigenschaften und verhindern als Haftverbesserer das Abtropfen und Verspritzen von Oel.

Die folgenden Beispiele erläutern die Herstellung der Aldosemercaptale dieser Erfindung näher.

Allgemeines Beispiel A:

Eine Aldose wird mit der doppelten Molmenge eines Mercaptans und dann mit so viel Trifluoressigsäure versetzt,dass etwa eine 50%-ige Lösung entsteht. Die klare Lösung wird bei Raumtemperatur stehen gelassen und der Reaktionsverlauf durch SH-Bestimmungen kontrolliert. Nach beendeter SH-Abnahme kann über die folgenden drei Wege $A_1$, $A_2$ und $A_3$ aufgearbeitet werden.

$A_1$ Das Reaktionsprodukt wird mit Wasser ausgefällt, der Festkörper abgesaugt und mehrfach mit Wasser gewaschen. Der Rückstand wird dann aus dem jeweiligen organischen Lösungsmittel umkristallisiert.

$A_2$ Das Reaktionsgemisch wird mit Diäthyläther versetzt; anschliessend wird bis zur Zweiphasigkeit Wasser zugegeben, die wässrige Phase abgetrennt und die organische Phase mit einer wässrigen Natriumbicarbonatlösung behandelt. Nach dem Trocknen über Natriumsulfat und einer Klärfiltration wird das Lösungsmittel am Rotationsverdampfer abdestilliert und der Rückstand aus dem jeweiligen organischen Lösungsmittel umkristallisiert.

$A_3$ Die Trifluoressigsäure wird am Rotationsverdampfer abdestilliert und der Rückstand aus dem jeweiligen organischen Lösungsmittel umkristallisiert.

Beispiel 1: 1. Version

36 Gew.-Teile D-(+)-Glucose, 31 Gew.-Teile 2-Mercaptoäthanol, 16 Gew.-Teile LEWASORB AC 10/H und 30 Gew.-Teile Wasser werden 8 bis 12 Stunden bei 45-50 °C gerührt. Der Reaktionsverlauf wird durch SH-Bestimmungen kontrolliert. Nach beendeter Reaktion wird das Gemisch mit 100 Gew.-Teilen $H_2O$ versetzt, das Harz durch Filtration abgetrennt, mit 100 Gew.-Teilen Wasser gewaschen und die vereinigten Filtrate am Rotationsverdampfer eingeengt. Der Rückstand von 57,8 g wird aus 150 ml Aethanol umkristallisiert.

Ausbeute: 28,3 Gew.-Teile ≙ 45% der theoretischen Ausbeute; weisse Kristalle, Smp. 84-86 °C

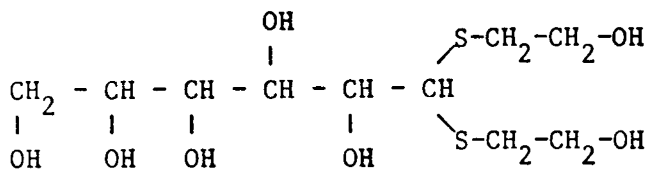

Beispiel 1: 2. Version

Ein Gemisch aus 200 g D-(+)-Glucose, 156 g 2-Mercaptoäthanol, 120 g LEWASORB AC 10/H und 150 g Methanol wird 6-12 Stunden unter Rühren auf 45-50 °C erwärmt. Nach beendeter Reaktion wird dem Reaktionsgemisch 400 ml Methanol zugesetzt, 10 Minuten stark gerührt und nach dem Absetzen des Festkörpers die überstehende Lösung abdekantiert. Dieser Vorgang wird nochmals mit 200 ml Methanol wiederholt. Die vereinigten methanolischen Lösungen werden am Rotationsverdampfer eingeengt und das nicht umgesetzte 2-Mercaptoäthanol mittels Oelpumpe abdestilliert.

Ausbeute: 294 g ≙ 92% der theoretischen Ausbeute.

Diese Verbindung wird wegen ihrer geringen Kristallisationsneigung vorteilhaft als ca. 50%ige Lösung in Wasser konfektioniert.

Mit dem im Reaktionskolben verbleibenden Katalysator kann die Reaktion wie oben beschrieben, noch mehrmals mit der gleichen Ausbeute wiederholt werden.

Beispiele 2-14: Analog dem in dem allgemeinen Beispiel A beschriebenen Verfahren werden weitere Verbindungen (Beispiele 2-6 und 8-14)hergestellt, die zusätzlich zu Beispiel 1 in der folgenden Tabelle I zusammengestellt sind.

Tabelle 1:

| Beispiel Nr. | Formel | Analytische Daten Schmelzpunkt [°C] | Hergestellt nach Beispiel | Bemerkungen: x% der theor. Ausbeute/(umkristallisiert in y)/weitere Eigenschaften |
|---|---|---|---|---|
| 1 | 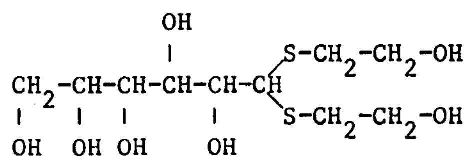 | 86 | | 45%/(Aethanol) |
| 2 | 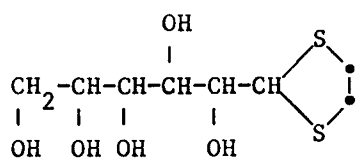 | 143 | $A_3$ | 52%/(Methanol) |
| 3 | 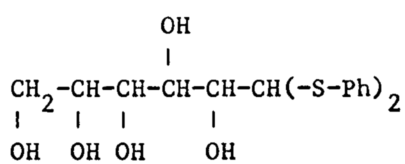 | 153 | $A_2$ | 30%/(i-Propanol) |

Tabelle 1: (Fortsetzung)

| Beispiel Nr. | Formel | Analytische Daten Schmelzpunkt [°C] | Hergestellt nach Beispiel | Bemerkungen: x% der theor. Ausbeute/ (umkristallisiert in y)/weitere Eigenschaften |
|---|---|---|---|---|
| 4 | 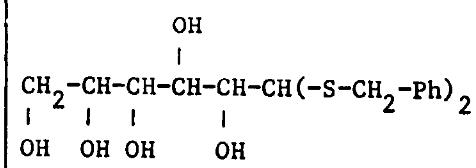 | 134 | $A_1$ | 73%/(i-Propanol) |
| 5 | 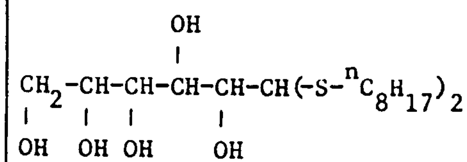 | 112 | $A_1$ | 65%/(i-Propanol) (bei 70°C in Oel löslich) |
| 6 | 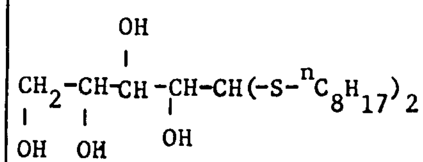 | Wachs/ Gel | $A_2$ | 91%/(bei RT in Oel löslich) |
| 7 | 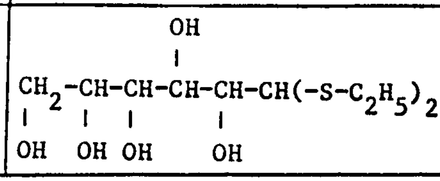 | 128 | käuflich (ev. nach $A_3$) | - |

Tabelle 1: (Fortsetzung)

| Beispiel Nr. | Formel | Analytische Daten Schmelzpunkt [°C] | Hergestellt nach Beispiel | Bemerkungen: x% der theor. Ausbeute/ (umkristallisiert in y)/weitere Eigenschaften |
|---|---|---|---|---|
| 8 | 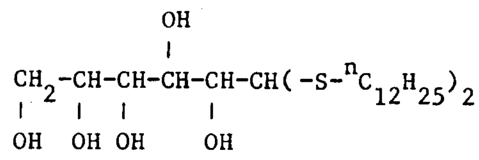 | 125 | $A_1$ | 89%/(i-Propanol) |
| 9 | 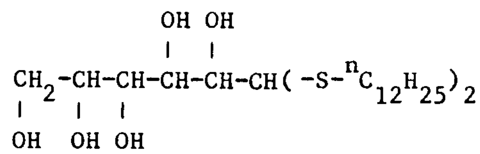 | 143 | $A_1$ | 56%/(i-Propanol) |
| 10 | 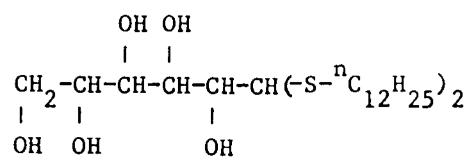 | 131 | $A_1$ | 64%/(i-Propanol) |
| 11 | 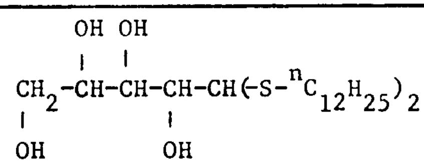 | 100 | $A_1$ | 63%/(i-Propanol) |

Tabelle 1: (Fortsetzung)

| Beispiel Nr. | Formel | Analytische Daten Schmelzpunkt [°C] | Hergestellt nach Beispiel | Bemerkungen: x% der theor. Ausbeute/ (unkristallisiert in y)/weitere Eigenschaften |
|---|---|---|---|---|
| 12 | OH OH<br>$CH_2$-CH-CH-CH-CH(-S-$^nC_{12}H_{25})_2$<br>OH OH | 101 | $A_1$ | 69%/(i-Propanol) |
| 13 | $CH_2$-CH-CH-CH-CH(-S-$^nC_{12}H_{25})_2$<br>OH OH OH OH | 96 | $A_2$ | 43%/(Essigester)/ (bei 60°C in Oel löslich) |
| 14 | OH<br>$CH_2$-CH-CH-CH-CH(-S-$^nC_{12}H_{25})_2$<br>OH OH OH | 87 | $A_2$ | 56%/(i-Propanol)/ (bei 50°C in Oel löslich) |
| 15 | OH CH$_3$<br>$CH_2$-CH-CH-CH-CH-CH(-S-$CH_2$-CH-$CH_2$-C-$CH_3)_2$<br>OH OH OH OH $CH_3$ $CH_3$ | 130-32 | $A_2$ | 43%/(i-Propanol) |

Die folgenden Anwendungsbeispiele dienen der näheren Erläuterung der anwendungstechnisch erhaltenen Ergebnisse; die Anwendungsbeispiele 1-3 beziehen sich auf Schmierstoffe und das Anwendungsbeispiel 4 auf Kunststoffe.

Anwendungsbeispiel 1: Mit dem Shell-Vierkugel-Apparat (IP 239/73 Extreme pressure and wear lubricant test for oils and greases-four ball-machine) werden folgende Werte bestimmt:

1. W.L. = Weld load (Schweisslast). Das ist die Last, bei der die 4 Kugeln innerhalb von 10 Sekunden zusammenschweissen.
2. W.S.D. = Wear Scar Diameter in mm: Das ist der mittlere Verschleissdurchmesser bei einer Belastung von 400 N während 10 Minuten.

Als Testflüssigkeit für die Wirksamkeit der Additive (Beispiel-Nr. 2 und 7) wird deionisiertes Wasser verwendet, welches als Rostinhibitor 0,75 Gew.% 2,4,6-Tris-(5'-carboxypentylamino)-1,3,5-triazin und so viel Triäthanolamin enthält, bis der pH-Wert von 8,5 erreicht wird. Als Testflüssigkeit für die Wirksamkeit des Additivs (Beispiel-Nr. 1) wird nur deionisiertes Wasser genommen, wobei der pH-Wert 9,4 beträgt.

Tabelle 2:

| Additiv: Beispiel-Nr. | 2,5% Additiv in der Testflüssigkeit | |
|---|---|---|
| | Test mit dem Shell-Vierkugel-Apparat | |
| | W.L. (N) | W.S.D. 10 Min. (mm) |
| 1 | 2250 | 0,75 |
| 2 | 1700 | 0,7 |
| 7 | 1450 | 1,05 |

Anwendungsbeispiel 2: Mit der Reibverschleisswaage nach Reichert (Reichert Wear Test DBGM 1749247) wird die Verschleisskalotte bestimmt.

Bei diesem Reibungsprüfgerät wird über ein Doppelhebelsystem eine fest eingespannte Prüfrolle an einen umlaufenden Schleifring angepresst, der mit seinem unteren Drittel in die zu prüfende Flüssigkeit taucht, deren Druckaufnahmevermögen beurteilt werden soll. Bei umlaufendem Schleifring entstehen je nach Druckaufnahmevermögen der Flüssigkeit auf die Prüfrolle Abschliffflächen (Verschleisskalotten), deren Grösse von der Tragfähigkeit des Prüfstoffes abhängt.

| Prüfbedingungen des Gerätes: | |
|---|---|
| Flüssigkeitsmenge: | ca. 25 ml |
| Prüfkörper: | Ring und Rolle, Achsen gekreuzt |
| Gleitgeschwindigkeit | 1,70 m/sec |
| Testdauer: | ≙ 100 Meter Laufstrecke |
| Ring und Rollenmaterial: | Stahl, gehärtet |
| Normallast: | 15 N Belastungsgewicht |
| Reibungsart: | Gleitreibung |
| Messgrössen: | Abriebfläche in $mm^2$ |

Als Testflüssigkeit für die Wirksamkeit der Additive (Beispiel-Nr. 2 und 7) wird deionisiertes Wasser verwendet, welches als Rostinhibitor 0,75 Gew.% 2,4,6-Tris(5'-carboxypentylamino)-1,3,5-triazin und so viel Triäthanolamin enthält, bis der pH-Wert von 8,5 erreicht wird. Als Testflüssigkeit für die Wirksamkeit des Additivs (Beispiel-Nr. 1) wird nur deionisiertes Wasser genommen, wobei der pH-Wert 9,4 beträgt.

Um die Wirksamkeit der Testflüssigkeiten aufzuzeigen, wird hier zum Vergleich der im Reichert-Test erzielte Wert für eine Referenzflüssigkeit, die aus 50% deionisiertem Wasser und 50% Aethanol besteht, angegeben; dieser Wert liegt im Bereich 28 bis 30 $mm^2$.

Tabelle 3

| Konzentration | 2,5% Additiv in der Testflüssigkeit | | | Testflüssigkeit ohne Additiv |
|---|---|---|---|---|
| Additiv Beispiel-Nr. | 1 | 2 | 7 | |
| Reichert<br>Wear Test<br>($mm^2$) | 15,0<br>14,5<br>14,3 | 8,7<br>8,7<br>8,0 | 11,7<br>13,2<br>13,2 | 28<br>28<br>30 |

Anwendungsbeispiel 3: Mit dem "Späne-Filterpapier-Verfahren" nach DIN 51360/Teil 2 werden die Korrosionseigenschaften bestimmt.

Bei diesem Verfahren wird in einem Becher mit Wasser nach bestimmten Vorschriften eine Mischung, die unter anderem auch das zu untersuchende Additiv enthält, hergestellt. Graugussspäne werden auf einem Rundfilter mit der frisch hergestellten Mischung benetzt, 2 Stunden lang in einer Petri-Schale der Raumtemperatur ausgesetzt und anschliessend das Rundfilter auf Korrosionserscheinungen visuell bewertet.

Die Korrosionsgrade werden aufgrund einer Tabelle (vgl. DIN 51360/Teil 2) ausgewertet, in welcher 0 keine und 1, 2, 3 und 4 zunehmend stärkere Korrosion bedeuten.

Als Testflüssigkeit für die Wirksamkeit der Additive (Beispiel-Nr. 2 und 7) wird deionisiertes Wasser verwendet, welches als Rostinhibitor 0,75 Gew.% 2,4,6-Tris-(5'-carboxypentylamino)-1,3,5-triazin und so viel Triäthanolamin enthält, bis der pH-Wert von 8,5 erreicht wird. Als Testflüssigkeit für die Wirksamkeit des Additivs (Beispiel-Nr. 1) wird nur deionisiertes Wasser genommen, wobei der pH-Wert 9,4 beträgt.

Tabelle 4

| Konzentration | 2,5% Additiv in der Testflüssigkeit | | |
|---|---|---|---|
| Additiv: Beispiel-Nr. | 1 | 2 | 7 |
| Korrosion | 0 | 0 | 0 |

Anwendungsbeispiel 4: Statischer Hitzetest

Um die thermostabilisierende Wirkung von bestimmten Additiven zu prüfen, wird ein Verfahren eingesetzt, bei dem die Farbänderung von Prüffolien bei konstanter höherer Temperatur in Abhängigkeit von der Zeit beurteilt.

Die Prüffolien werden nach der folgenden Rezeptur hergestellt:

| | |
|---|---|
| SOLVIC 246 GA | 100,00 Teile |
| REOPLAST 39 | 3,00 Teile |
| Ca-Stearat | 0,35 Teile |
| Zn-Stearat | 0,15 Teile |
| IRGASTAB CH 300 | 0,55 Teile |
| Testsubstanz | x Teile ≙ 2,5 mmol |

Die Herstellung der Prüffolie erfolgt auf einem Mischwalzwerk bei 180 °C, wobei die Folie eine Stärke von 0,3 mm aufweist.

Dann werden Folienabschnitte gleicher Abmessungen in einem Wärmeschrank bei konstanter Temperatur, 180 °C, gelagert. Nach festgelegten Zeitabschnitten (vgl. Tabelle 5) wird ein Muster zur Beurteilung der Farbveränderung entnommen. Die Prüfung wird so lange fortgesetzt, bis ein deutlicher Umschlag nach dunkleren Farbtönen eingetreten ist. Durch optischen Vergleich lassen sich so Rückschlüsse auf die Wirksamkeit der Stabilisatoren ziehen.

Der optische Vergleich wird durch Messung des Yellowness-Index nach DIN 6167 (ASTM D 1925-70) vorgenommen.

Tabelle 5:

| Testsubstanz Beispiel Nr. (vgl. Tabelle 1) | x Teile Testsubstanz in der Prüffolie | Yellowness - Index - Bestimmung: Prüffolie ohne Testsubstanz | Prüffolie mit Testsubstanz nach y Minuten: 0 min | 5 min | 10 min | 15 min | 20 min | 25 min | 30 min | 35 min | 40 min | 45 min |
|---|---|---|---|---|---|---|---|---|---|---|---|---|
| 2 | 0,64(≙2,5mmol) | 25,8 | 10,5 | 16,1 | 20,8 | 24,9 | 38,6 | 56,3 | 90,1 | 96,9 | 117,0 | 124,3 |
| 3 | 0,96(≙2,5mmol) | 23,9 | 9,4 | 12,7 | 16,0 | 20,2 | 19,6 | 28,8 | 36,3 | 42,3 | 58,1 | 90,0 |
| 6 | 1,14(≙2,5mmol) | 18,0 | 12,0 | 16,7 | 22,3 | 25,6 | 28,2 | 34,1 | 35,9 | 50,9 | 48,4 | 81,7 |

## Patentansprüche

1. Zusammensetzungen enthaltend
   A) einen Schmierstoff, einen Kunststoff oder eine Hydraulikflüssigkeit,

B) eine Verbindung der Formel I

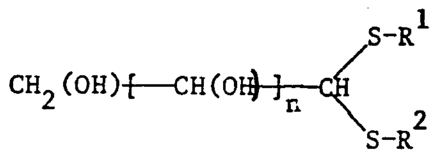

(I),

worin n eine natürliche Zahl von 2 bis 6 sein kann und worin $R^1$ und $R^2$ gleich oder verschieden sind und jeweils unsubstituiertes, substituiertes oder durch -O- oder -S- unterbrochenes $C_1$-$C_{18}$ Alkyl, $-(CH_2)_r$-CO-N($C_1$-$C_{17}$ Alkyl)$_2$ mit r gleich 1 oder 2, Phenyl, Benzyl oder $-(CH_2)_r$-CO-O-$R^3$ bedeuten, wobei r gleich 1 oder 2 sein kann und $R^3$ Alkalimetall oder $C_1$-$C_{14}$ Alkyl darstellt, ferner worin $R^1$ und $R^2$ $-CH_2$-CH(OH)-$R^4$ sind, wobei $R^4$ Wasserstoff, unsubstituiertes oder durch -OH substituiertes $C_1$-$C_{16}$ Alkyl oder $-CH_2$-Y-($C_1$-$C_{15}$ Alkyl) mit Y gleich -O- oder -S- ist, oder worin $R^1$ und $R^2$ zusammen $-(CH_2)_m$- bilden, wobei m eine natürliche Zahl von 2 bis 4 sein kann.

**2.** Zusammensetzungen gemäss Anspruch 1, enthaltend eine Verbindung der Formel I, worin n eine natürliche Zahl von 3 bis 6 sein kann, und worin $R^1$ und $R^2$ gleich sind und unsubstituiertes oder durch -O- oder -S- unterbrochenes $C_1$-$C_{16}$ Alkyl, Phenyl, Benzyl oder $-(CH_2)_r$-CO-O-$R^3$bedeuten, wobei r gleich 1 oder 2 sein kann und $R^3$ Alkalimetall ist, oder worin $R^1$ und $R^2$ $-CH_2$-CH(OH)-$R^4$ sind, wobei $R^4$ Wasserstoff oder $C_1$-$C_{14}$ Alkyl oder $-CH_2$-O-($C_1$-$C_{13}$ Alkyl) ist, oder worin $R^1$ und $R^2$ zusammen $-(CH_2)_m$- bilden, wobei m eine natürliche Zahl von 2 bis 4 sein kann.

**3.** Zusammensetzungen gemäss Anspruch 2, enthaltend eine Verbindung der Formel I, worin n eine natürliche Zahl von 3 bis 6 sein kann, und worin $R^1$ und $R^2$ gleich sind und $C_8$-$C_{16}$ Alkyl oder $-CH_2$-O-($C_7$-$C_{15}$ Alkyl), Phenyl, Benzyl oder $-CH_2$-COOK, $-CH_2$-COONa oder $-CH_2$-$CH_2$-COOK bedeuten oder worin $R^1$ und $R^2$ $-CH_2$-CH(OH)-$R^4$sind, wobei $R^4$ Wasserstoff oder $C_6$-$C_{14}$ Alkyl oder $-CH_2$-O-($C_5$-$C_{13}$ Alkyl) ist, oder worin $R^1$ und $R^2$ zusammen $-(CH_2)_m$- bilden, wobei m eine natürliche Zahl von 2 bis 4 sein kann.

**4.** Verbindungen der Formel I nach Anspruch 1, worin n eine natürliche Zahl von 2 bis 6 sein kann, und worin $R^1$ und $R^2$ gleich oder verschieden sind und jeweils verzweigtes $C_5$-$C_{18}$ Alkyl, $-(CH_2)_r$-CO-O-$R^3$, wobei r gleich 1 oder 2 sein kann und $R^3$ Alkalimetall oder $C_1$-$C_{14}$-Alkyl darstellt, oder $-CH_2$-CH(OH)-$R^4$ bedeuten, wobei $R^4$ Wasserstoff oder unsubstituiertes oder durch -OH substituiertes $C_1$-$C_{16}$ Alkyl oder $-CH_2$-Y-($C_1$-$C_{15}$ Alkyl) mit Y gleich -O- oder -S- darstellt.

**5.** Verbindungen der Formel I nach Anspruch 1, gemäss Anspruch 4, worin n eine natürliche Zahl von 2 bis 6 sein kann, und worin $R^1$ und $R^2$ gleich oder verschieden sind und jeweils $-(CH_2)_r$-CO-O-$R^3$, wobei r gleich 1 oder 2 sein kann und $R^3$ Alkalimetall oder $C_1$-$C_{14}$ Alkyl darstellt, oder $-CH_2$-CH(OH)-$R^4$ bedeuten, wobei $R^4$ Wasserstoff oder unsubstituiertes oder durch -OH substituiertes $C_1$-$C_{16}$ Alkyl oder $-CH_2$-Y-($C_1$-$C_{15}$ Alkyl) mit Y gleich -O- oder -S- ist.

**6.** Verbindungen der Formel I nach Anspruch 1 gemäss Anspruch 4, worin n eine natürliche Zahl von 3 bis 6 sein kann, und worin $R^1$ und $R^2$ gleich sind und $-CH_2$-CH-[$(CH_2)_q$-$CH_3$][$(CH_2)_{q+2}$-$CH_3$], wobei q gleich 1 bis 5 sein kann, $-(CH_2)_r$-CO-O-$R^3$, wobei r gleich 1 oder 2 sein kann und $R^3$ Alkalimetall ist, oder $-CH_2$-CH(OH)-$R^4$ bedeuten, wobei $R^4$ Wasserstoff oder $C_1$-$C_{14}$ Alkyl oder $-CH_2$-O-($C_1$-$C_{13}$ Alkyl) bedeutet.

**7.** Verbindungen der Formel I nach Anspruch 1 gemäss Anspruch 6, worin n eine natürliche Zahl von 4 bis 6 sein kann, und worin $R^1$ und $R^2$ gleich sind und -2-Ethyl-hexyl, -2-Butyl-octyl,-2-Hexyl-decyl, $-CH_2$-COOK, $-CH_2$-COONa oder $-CH_2$-$CH_2$-COOK bedeuten oder worin $R^1$ und $R^2$ $-CH_2$-CH(OH)-$R^4$ sind, wobei $R^4$ Wasserstoff oder $C_6$-$C_{14}$ Alkyl oder $-CH_2$-O-($C_5$-$C_{13}$ Alkyl) darstellt.

**8.** Verwendung der Verbindungen der Formel I gemäss Anspruch 1 als Stabilisatoren für thermischem und/oder oxidativem Abbau unterliegendes organisches Material.

**9.** Verwendung gemäss Anspruch 8 für Schmierstoffe.

**10.** Verwendung gemäss Anspruch 8 für Hydraulik-Flüssigkeiten.

**11.** Verwendung gemäss Anspruch 8 für Kunststoffe.

## Claims

**1.** A composition containing
A) a lubricant, a plastic or a hydraulic fluid,
B) a compound of the formula I

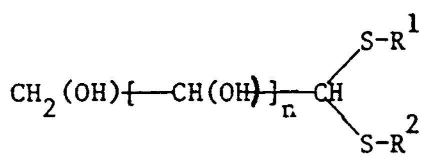

(I)

wherein n can be an integer from 2 to 6, and wherein $R^1$ and $R^2$ are identical or different and in each case are $C_1$-$C_{18}$alkyl which is unsubstituted, substituted or interrupted by -O- or -S-, or are $-(CH_2)_r$-CO-N($C_1$-$C_{17}$alkyl)$_2$, r being 1 or 2, or are phenyl, benzyl or $-(CH_2)_r$-CO-O-$R^3$, in which r can be 1 or 2 and $R^3$ is an alkali metal or $C_1$-$C_{14}$alkyl, wherein furthermore $R^1$ and $R^2$ are $-CH_2$-CH(OH)-$R^4$, in which $R^4$ is hydrogen or $C_1$-$C_{16}$alkyl which is unsubstituted or substituted by -OH, or $-CH_2$-Y-($C_1$-$C_{15}$alkyl), in which Y is -O- or -S-, or wherein $R^1$ and $R^2$ together form $-(CH_2)_m$- in which m can be an integer from 2 to 4.

**2.** A composition according to claim 1, containing a compound of the formula I wherein n can be an integer from 3 to 6, and wherein $R^1$ and $R^2$ are identical and are $C_1$-$C_{16}$alkyl which is unsubstituted or interrupted by -O- or -S- or are phenyl, benzyl or $-(CH_2)_r$-CO-O-$R^3$, in which r can be 1 or 2 and $R^3$ is an alkali metal, or wherein $R^1$ and $R^2$ are $-CH_2$-CH(OH)-$R^4$, in which $R^4$ is hydrogen or $C_1$-$C_{14}$alkyl or $-CH_2$-O-($C_1$-$C_{13}$alkyl), or wherein $R^1$ and $R^2$ together form $-(CH_2)_m$- in which m can be an integer from 2 to 4.

**3.** A composition according to claim 2, containing a compound of the formula I wherein n can be an integer from 3 to 6, and wherein $R^1$ and $R^2$ are identical and are $C_8$-$C_{16}$alkyl or $-CH_2$-O-($C_7$-$C_{15}$alkyl), phenyl, benzyl or $-CH_2$-COOK, $-CN_2$-COONa or $-CH_2$-$CH_2$-COOK, or wherein $R^1$ and $R^2$ are $-CH_2$-CH-(OH)-$R^4$, in which $R^4$ is hydrogen or $C_6$-$C_{14}$alkyl or $-CH_2$-O-($C_5$-$C_{13}$alkyl), or wherein $R^1$ and $R^2$ together form $-(CH_2)_m$- in which m can be an integer from 2 to 4.

**4.** A compound of the formula I according to claim 1, wherein n can be an integer from 2 to 6, and wherein $R^1$ and $R^2$ are identical or different and in each case are branched $C_5$-$C_{18}$alkyl, $-(CH_2)_r$-CO-O-$R^3$, in which r can be 1 or 2 and $R^3$ is an alkali metal or $C_1$-$C_{14}$alkyl, or are $-CH_2$-CH(OH)-$R^4$, in which $R^4$ is hydrogen or $C_1$-$C_{16}$alkyl which is unsubstituted or substituted by -OH, or is $-CH_2$-Y-($C_1$-$C_{15}$alkyl) in which Y is -O- or -S-.

**5.** A compound according to claim 4 of the formula I according to claim 1, wherein n can be an integer from 2 to 6, and wherein $R^1$ and $R^2$ are identical or different and in which case are $-(CH_2)_r$-CO-O-$R^3$, in which r can be 1 or 2 and $R^3$ is an alkali metal or $C_1$-$C_{14}$alkyl, or are $-CH_2$-CH(OH)-$R^4$, in which $R^4$ is hydrogen or $C_1$-$C_{16}$alkyl which is unsubstituted or substituted by -OH, or is $-CH_2$-Y-($C_1$-$C_{15}$alkyl) in which Y is -O- or -S-.

**6.** A compound according to claim 4 of the formula I according to claim 1, wherein n can be an integer from 3 to 6, and wherein $R^1$ and $R^2$ are identical and are $-CH_2$-CH-[$(CH_2)_q$-$CH_3$][$(CH_2)_{q+2}$-$CH_3$], in which q can be 1 to 5, or are $-(CH_2)_r$-CO-O-$R^3$, in which r can be 1 or 2 and $R^3$ is an alkali metal, or are $-CH_2$-CH(OH)-$R^4$ in which $R^4$ is hydrogen or $C_1$-$C_{14}$alkyl or $-CH_2$-O-($C_1$-$C_{13}$alkyl).

**7.** A compound according to claim 6 of the formula I according to claim 1, wherein n can be an integer from 4 to 6, and wherein $R^1$ and $R^2$ are identical and are -2-ethylhexyl, -2-butyloctyl, -2-hexyldecyl, $-CH_2$-COOK, $-CH_2$-COONa or $-CH_2$-$CH_2$-COOK, or wherein $R^1$ and $R^2$ are $-CH_2$-CH(OH)-$R^4$ in which $R^4$

is hydrogen or $C_6$-$C_{14}$alkyl or -$CH_2$-O-($C_5$-$C_{13}$alkyl).

**8.** The use of a compound of the formula I according to claim 1 as a stabiliser for organic material exposed to thermal and/or oxidative degradation.

**9.** The use according to claim 8 for lubricants.

**10.** The use according to claim 8 for hydraulic fluids.

**11.** The use according to claim 8 for plastics.

**Revendications**

**1.** Compositions contenant :
A) un lubrifiant, une matière plastique ou un liquide hydraulique,
B) un composé de formule I

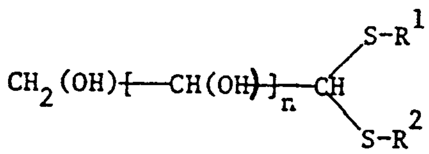

(I),

dans laquelle n peut être un nombre entier valant 2 à 6, et $R^1$ et $R^2$ sont identiques ou différents et représentent chacun un groupe alkyle en $C_1$ à C18, non substitué, substitué ou interrompu par -O- ou -S-, un groupe -$(CH_2)_r$-CO-N(alkyle en $C_1$ à $C_{17})_2$ avec R valant 1 ou 2, un groupe phényle, benzyle ou -$(CH_2)_r$-CO-0-$R^3$, r pouvant valoir 1 ou 2 , et $R^3$ représente un métal alcalin ou un groupe alkyle en $C_1$ à $C_{14}$; $R^1$ et $R^2$ peuvent en outre représenter $CH_2$--CH(OH)-$R^4$, où $R^4$ représente un atome d'hydrogène, un groupe alkyle en $C_1$ à $C_{16}$ non substitué ou substitué par OH, ou $CH_2$-Y-(alkyle en $C_1$ à $C_{15}$), dans lequel Y représente -O- ou -S-, ou bien $R^1$ et $R^2$ forment ensemble -$(CH_2)_m$-, m pouvant être un nombre entier valant 2 à 4.

**2.** Compositions selon la revendication 1, contenant un composé de formule 1, dans laquelle n peut être un nombre entier valant 3 à 6, et $R^1$ et $R^2$ sont identiques et représentent chacun un groupe alkyle en $C_1$ à $C_{16}$ non substitué ou interrompu par -O- ou -S-, un groupe phényle, benzyle ou -$(CH_2)_r$-CO-O-$R^3$, r pouvant valoir 1 ou 2 et $R^4$ représentant un métal alcalin, ou bien $R^1$ et $R^2$ représentent -$CH_2$-CH(OH)-$R^4$, et $R^4$ représente un atome d'hydrogène ou un groupe alkyle en $C_1$ à $C_{14}$ ou -$CH_2$-O-(alkyle en $C_1$ à $C_{13}$), ou bien $R^1$ et $R^2$ forment ensemble -$(CH_2)_m$ -, m pouvant être un nombre entier valant 2 à 4.

**3.** Compositions selon la revendication 2, contenant un composé de formule I, dans laquelle N peut être un nombre entier valant 3 à 6, et $R^1$ et $R^2$ sont identiques et représentent chacun un groupe alkyle en $C_8$ à $C_{16}$ ou un groupe -$CH_2$-O-(alkyle en $C_7$ à $C_{15}$), phényle, benzyle ou -$CH_2$-COOK, -$CH_2$-COONa ou -$CH_2$-$CH_2$-COOK, ou bien $R^1$ et $R^2$ représentent un -$CH_2$-CH(OH)-$R^4$, et $R^4$ représente un atome d'hydrogène ou un groupe alkyle en $C_6$ à $C_{14}$ ou -$CH_2$-O-(alkyle en $C_5$ à $C_{13}$), ou bien $R^1$ et $R^2$ forment ensemble -$(CH_2)$m-, m pouvant être un nombre entier valant 2 à 4.

**4.** Composés selon la revendication 1, dans laquelle n peut être un nombre entier valant 2 à 6, et $R^1$ et $R^2$ sont identiques ou différents et représentent chacun un groupe alkyle ramifié en $C_5$ à $C_{18}$, -$(CH_2)$-$_r$-CO-O-$R^3$, r pouvant valoir 1 ou 2 et $R^3$ étant un métal alcalin ou représentant un groupe alkyle en $C_1$ à $C_{14}$, ou bien $R^1$ et $R^2$ représentent -$CH_2$-CH(OH)-$R^4$, et $R^4$ représente un atome d'hydrogène ou un groupe alkyle en $C_1$ à $C_{16}$, non substitué ou substitué par -OH ou un groupe -$CH_2$-H-(alkyle en $C_1$ à $C_{15}$),dans lequel Y représente -O-ou S .

**5.** Composés de formule I selon la revendication 1 ou selon la revendication 4 dans lesquelles n peut être un nombre entier valant 2 à 6, et les symboles $R^1$ et $R^2$ sont identiques ou différents et représentent chacun -$(CH_2)_r$-CO-O-$R^3$, où r peut valoir 1 ou 2 et $R^3$ représente un métal alcalin ou un groupe alkyle en $C_1$ à $C_{14}$, ou bien $R^1$ et $R^2$ représentent -$CH_2$-CH(OH)-$R^4$, et $R^4$ représente un atome d'hydrogène

ou un groupe alkyle en $C_1$ à $C_{16}$, non substitué ou substitué par -OH, ou un groupe $-CH_2$-Y-(alkyle en $C_1$ à $C_{15}$) dans lequel Y représente -O- ou -S-.

**6.** Composés de formule I selon la revendication 1, selon la revendication 4, dans lequelles n peut être un nombre entier valant 3 à 6, et $R^1$ et $R^2$ sont identiques et représentent chacun $-CH_2-CH-[(CH_2)_q-CH_3][-(CH_2)_{q+2}-CH_3]$, où q peut valoir 1 à 5, $-(CH_2)_r-CO-O-R^3$, r peut valoir 1 ou 2 et $R^3$ représente un métal alcalin ou bien $-CH_2-CH(OH)-R^4$, où $R^4$ représente un atome d'hydrogène ou un groupe alkyle en $C_1$ à $C_{14}$ ou $-CH_2$-O-(alkyle en $C_1$ à $C_{13}$).

**7.** Composés de formule 1 de la revendication 1 selon la revendication 6, dans lesquelles n peut être un nombre entier valant 4 à 6, et $R^1$ et $R^2$ sont identiques et représentent chacun un groupe -2-éthyl-hexyle, -2-butyl-octyle, -2-hexyl-décyle, $-CH_2-COOK$, $-CH_2-COONa$ ou $-CH_2-CH_2-COOK$, ou bien $R^1$ et $R^2$ représentent $-CH_2-CH(OH)-R^4$ et $R^4$ représente un atome d'hydrogène ou un groupe alkyle en $C_6$ à $C_{14}$ ou $-CH_2$-O-(alkyle en $C_5$ à $C_{13}$).

**8.** Utilisation des composés de formule I selon la revendication 1, comme stabilisant pour empêcher une dégradation thermique et/ou par oxydation d'un matériau organique.

**9.** Utilisation selon la revendication 8 pour des lubrifiants.

**10.** Utilisation selon la revendication 8 pour des fluides hydrauliques.

**11.** Utilisation selon la revendication 8 pour des matières synthétiques ou plastiques.